# EUROPÄISCHE PATENTSCHRIFT

(11) **EP 1 163 884 B1**
(45) Veröffentlichungstag und Bekanntmachung des Hinweises auf die Patenterteilung: **30.09.2009**
(21) Anmeldenummer: 01113411.1
(22) Anmeldetag: 01.06.2001
(51) Int. Cl.: A61B 17/22

(54) **Vorrichtung zum Entfernen von Körpersteinen mit einem intrakorporalen Lithotripter**
Intracorporeal lithotripter for removing calculi
Lithotripteur intracorporelle pour évacuer des concrétions calcaires

(30) Priorität: 15.06.2000 DE 10029582
(43) Veröffentlichungstag der Anmeldung: 19.12.2001
(73) Patentinhaber: Ferton Holding SA, 2800 Delemont (CH)
(72) Erfinder: Menne, Andreas, Dr., 88709 Meersburg (DE); Merkle, Wolfgang, 88709 Meersburg (DE)
(74) Vertreter: Müller, Frank Peter

(56) Entgegenhaltungen:
- EP-A- 0 421 285
- DE-A- 3 625 749
- US-A- 4 827 911
- US-A- 4 903 696
- US-A- 5 807 307

## Beschreibung

Die Erfindung bezieht sich auf eine Vorrichtung zum Entfernen von Körpersteinen mit einem intrakorporalen Lithotripter gemäß dem Oberbegriff des Anspruches 1.

Für die Entfernung von Körpersteinen aus Körperhöhlen ist es bei Überschreitung einer für einen natürlichen Abgang noch ausreichenden Steingröße regelmäßig erforderlich, die Körpersteine zunächst zu zerkleinern, wobei die Zerkleinerung in kleine, spontan abgangsfähige oder direkt aus dem Körper ausspülbare Partikel vorgenommen wird. Die Zerkleinerung der Körpersteine wird dabei durch mechanische Druck- und Zugspannungen vorgenommen, die bei der intrakorporalen Lithotripsie mit dem distalen Ende einer als Wellenleiter dienenden Metallsonde auf die Körpersteine ausgeübt werden. Solche Spannungen führen zu einem Absprengen von Fragmenten aus der Oberfläche eines Steines und bewirken schließlich dessen Zertrümmerung. Für diese Steinzertrümmerung existiert allgemein das Problem einer geeigneten Energieübertragung mit besonderer Beachtung einer Vermeidung von Nebeneffekten auf das biologische Gewebe, das für die Steinzertrümmerung daher nicht als ein Widerlager dienen sollte.

Zur Durchführung einer intrakorporalen Lithotripsie ist aus der EP 0 421 285 B1 eine Vorrichtung bekannt, bei welcher eine Metallsonde oder Sonotrode durch einen elektrisch angesteuerten Ultraschallwandler zu longitudinalen Schwingungen angeregt wird. Mit dem distalen Ende der in den Arbeitskanal eines Endoskops eingesetzten Sonde kann daher die Zertrümmerung eines Körpersteins veranlaßt werden. Der Ultraschallwandler ist dabei mit zwei piezokeramischen Scheiben ausgebildet, die zwischen einem Reflektor und einem Horn verspannt sind. Die beiden Scheiben werden für eine periodische Schwingungsanregung der Sonotrode durch eine Schaltungsanordnung angesteuert, die aus einem spannungsgesteuerten Oszillator besteht, dessen Ausgangssignal über einen Ausgangsverstärker und einen Ausgangsübertrager an die beiden piezokeramischen Scheiben angeliefert wird. Die Schaltungsanordnung umfaßt daneben noch einen Phasenkomparator, der die Phasen der Ausgangsspannung und des Ausgangsstroms des Ausgangsübertragers vergleicht sowie eine Regelspannung zur Steuerung des Oszillators erzeugt. Mit einer Vorrichtung dieser Ausbildung lassen sich die Körpersteine in aller Regel zu sehr feinen Fragmenten zertrümmern. Für die Feinfragmente wird dabei eine Partikelgröße erhalten, die ein meistens problemloses Absaugen durch einen axialen Hohlraum der Sonde hindurch bis hin zu ihrem proximalen Ende erlaubt, an welchem ein durch den Ultraschallwandler hindurchgeführter Sauganschluß zur Wirkung gebracht ist. Die Steinzertrümmerung mittels solcher Ultraschall-Lithotripter ist jedoch relativ zeitaufwändig, da sich mit dem distalen Ende der hohlen Sonotrode bei den für eine gewebeschonende Steinbearbeitung üblichen Ultraschallfrequenzen von etwa 20 bis 25 kHz und Amplituden der Sondenspitze bis etwa 50 µm die Steinzertrümmerung nur reichlich langsam fortführen läßt. Erschwerungen ergeben sich dabei auch bei den härteren Körpersteinen, die sich bei den Ultraschallfrequenzen und Amplituden dieser Größenordnung nicht spontan abschaben lassen, sodaß ihre Bearbeitung entweder sehr lange dauert oder überhaupt nicht möglich ist.

Aus der EP 0 317 507 B1 ist ein Lithotripter bekannt, bei welchem das proximale Ende einer Metallsonde mit einem pneumatisch angetriebenen Schlagteil beaufschlagt wird, um mit einer dadurch erhaltenen Stoßenergie eine die Metallsonde bis hin zu ihrem distalen Ende durchlaufende Stoß- bzw. Druckwelle zu erzeugen. Mit dieser Stoß- bzw. Druckwelle wird auf einen Körperstein eingewirkt, wobei mit der Sondenspitze eine Berührung mit den Steinen eingehalten wird. Solche Stoßwellen-Lithotripter, die bei anderen Ausführungen auch einen elektrischen Antrieb des Schlagteils aufweisen können, ergeben allgemein einen relativ einfachen Geräteaufbau bei ebenfalls sehr guten Fragmentierungsleistungen auch an härteren Körpersteinen. Bei diesen Lithotriptern muß jedoch die Steinzertrümmerung bis hin zu einer absaugfähigen Partikelgröße immer noch relativ zeitaufwendig fortgeführt werden.

Die US-4,827,911 offenbart eine Vorrichtung und ein Verfahren zum Entfernen von Körpersteinen, bei welchem ein an einen Generator anschließbares piezoelektrisches Wandlerelement eine Sonotrode in periodische Schwingungen versetzt.

Der Erfindung liegt die Aufgabe zugrunde, eine Vorrichtung der eingangs genannten Art derart auszubilden, daß damit unter Berücksichtigung der Vor- und Nachteile dieser bekannten Verfahren eine flexiblere Steinzertrümmerung durchführbar ist.

Diese Aufgabe wird erfindungsgemäß gelöst mit einer Vorrichtung der durch den Anspruch 1 angegebenen Ausbildung, die mit den Merkmalen der weiteren Ansprüche eine vorteilhafte Gestaltungsmöglichkeit erfährt.

Die erfindungsgemäße Vorrichtung verfügt somit über einen ersten Anwendungsmodus, bei welchem eine periodische Schwingung der Metallsonde bzw. Sonotrode im wesentlichen wie bei den bisher bekannten Ultraschall-Lithotriptern durch die angeschlossene Schaltungsanordnung zur Ausbildung einer stehenden Welle veranlaßt wird, die eine sinusförmige Bewegung der Sondenspitze in axialer Richtung ergibt. Für eine Steinzertrümmerung kann dabei mit der Sondenspitze auf die Steinoberfläche mechanisch eingewirkt werden. Bei einer impulsförmigen Schwingungsanregung der Metallsonde bzw. Sonotrode gemäß einem zweiten Anwendungsmodus der Vorrichtung, auf welchen mit einem einfachen Umschalter umgeschaltet werden kann, wird dagegen der Ultraschallwandler mit einem einzigen Spannungsimpuls für die Bereitstellung einer Ausgangsleistung angesteuert, welche die wenigstens eine piezokeramische Scheibe plötzlich ausdehnen läßt, sodaß in der mit dem Horn verbundenen Metallsonde bzw. Sonotrode eine Druckwelle erzeugt wird, die an der Sondenspitze einen in den Körperstein eingeleiteten Druckimpuls erzeugt. Da ein solcher Druckimpuls eine einmalige Belastung der Sonotrode in einem relativ kleinen Zeitfenster ergibt, kann bei dieser impulsförmigen Schwingungsanregung über die Anschlußverbindung an den piezoelektrischen Wandler eine wesentlich höhere Leistung angeliefert werden als bei der periodischen Schwingungsanregung, sodaß bei diesem zweiten Anwendungsmodus Ergebnisse für die Steinzertrümmerung erzielbar sind, die vergleichbar mit denjenigen bei den bekannten Stoßwellen-Lithotriptern sind.

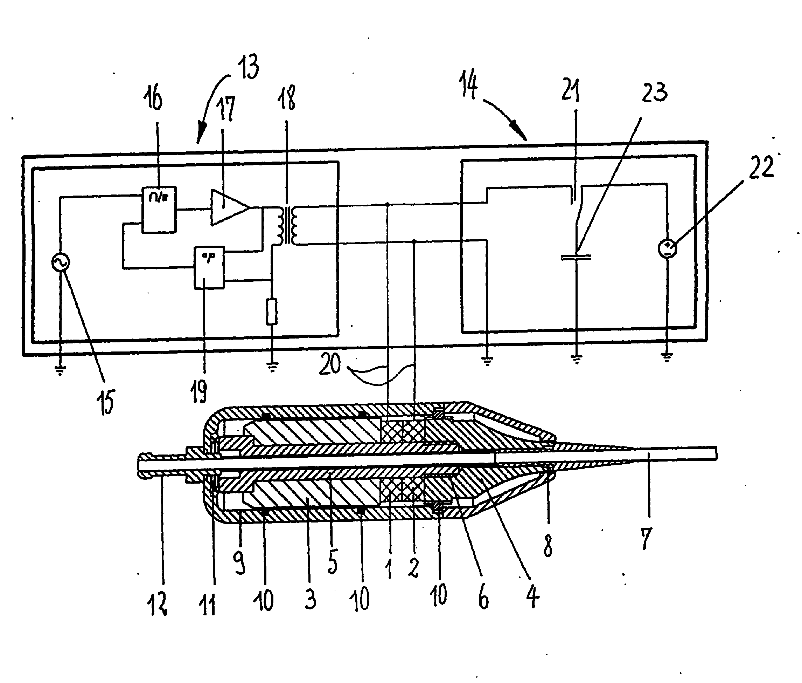

Ein Ausführungsbeispiel der erfindungsgemäßen Vorrichtung ist in der Zeichnung schematisch dargestellt und wird nachfolgend näher beschrieben.

Die erfindungsgemäße Vorrichtung besteht aus einem Ultraschallwandler, der als ein piezoelektrischer Wandler mit zwei piezokeramischen Scheiben 1 und 2 mit einer Anordnung zwischen einem Reflektor 3 und einem Horn 4 ausgebildet ist. Die aus diesen Bauteilen bestehende Anordnung des Ultraschallwandlers wird durch eine hohle Spannschraube 5 zusammengehalten, welche die beiden keramischen Scheiben 1, 2 und den Reflektor 3 axial durchsetzt und mit dem Horn 4 über ein Schraubgewinde 6 verschraubt ist. Mit dem Horn 4 ist andererseits eine rohrförmige Metallsonde bzw. Sonotrode 7 an einem Schraubansatz 8 verschraubt, sodaß sich der Hohlraum der Spannschraube 5 in dem Hohlraum der Metallsonde bzw. Sonotrode 7 axial fortsetzt.

Die mit der Spannschraube 5 zusammengehaltene und gegeneinander verspannte Anordnung der einzelnen Bauteile des Ultraschallwandlers ist innerhalb eines Gehäuses 9 untergebracht. Durch eine zweiteilige Ausbildung dieses Gehäuses mit einer Teilungsebene im Bereich des Horns 4 ist dabei mit der Spannschraube 5 auch eine elastische Verspannung gegen das Gehäuse 9 erreicht, die durch elastische Stützmittel in der Ausbildung von O-Ringen 10 und einer Dichtmanschette 11 eine Abstützung erfährt. Mit der hohlen Spannschraube 5 ist ein äußerer Sauganschluß 12 verbunden, sodaß die Steinfragmente, die bei einer Steinzertrümmerung am distalen Ende der Metallsonde 7 anfallen, über den Hohlraum der Metallsonde 7 und den damit axial fluchtenden Hohlraum der Spannschraube 5 spontan abgesaugt werden können.

Die beiden piezokeramischen Scheiben 1, 2 sind für eine an die Metallsonde 7 weitergeleitete Schwingungserzeugung mit einer elektrischen Ansteuerung verbunden, die mit einer ersten Schaltungsanordnung 13 und einer davon getrennten zweiten Schaltungsanordnung 14 ausgebildet ist. Die Schaltungsanordnung 13 ist für eine periodische Schwingungsanregung der Metallsonde vorgesehen und ist dafür im wesentlichen mit einem spannungsgesteuerten Oszillator 15 ausgebildet, der über einen U/F-Wandler 16 und einen nachgeschalteten Verstärker 17 mit einem Impedanz-Transformator 18 verbunden ist. Der Impedanz-Transformator 18 ist mit dem U/F-Wandler 16 über einen Phasenkomparator 19 rückgekoppelt, welcher die Phasen der Ausgangsspannung und des Ausgangsstroms des Impedanz-Transformators 18 vergleicht und eine Regelspannung zur Steuerung des Oszillators 15 erzeugt. Daher wird ein für die periodische Schwingunsanregung der Metallsonde 7 über die Anschlußverbindung 20 ein passendes Ausgangssignal an die beiden piezokeramischen Scheiben 1, 2 angeliefert.

Die piezokeramischen Scheiben 1, 2 sind daneben mit der zweiten Schaltungsanordnung 14 verbunden, durch welche eine impulsförmige Schwingungsanregung der Metallsonde 7 erhalten wird. Die Schaltungsanordnung 14 besteht aus einer Spannungsquelle 22 und einem Kondensator 23, der in einer ersten Schaltstellung eines Umschalters 21 durch die Spannungsquelle 22 aufgeladen wird. Der Kondensator 23 wird in einer zweiten Schalterstellung des Umschalters 21 entladen, sodaß eine impulsförmige Schwingungsanregung der Metallsonde erhalten wird. Die Kondensatorladung wird dabei einmalig über die Anschlußverbindung 20 an die beiden piezokeramischen Scheiben 1, 2 weitergeleitet, wobei durch das Ausgangssignal der Schaltungsanordnung 14 eine wesentlich höhere Leistung an den piezoelektrischen Wandler angeliefert werden kann als mit dem Ausgangssignal der für die periodische Schwingungsanregung maßgeblichen Schaltungsanordnung 13. Mit dieser höheren Leistung wird dabei eine plötzliche Ausdehnung der beiden piezokeramischen Scheiben 1,2 erhalten, die zu einer einmaligen Druckwellenerzeugung in der Metallsonde 7 und damit zu der Abgabe eines Druckimpulses an dem distalen Ende der Sonde führt. Dieser Druckimpuls wird unmittelbar in einen Körperstein eingeleitet, sodaß der Stein augenblicklich zertrümmert wird.

Um für die impulsförmige Schwingungsanregung der Metallsonde optimale Ergebnisse zu erhalten, sollte das Horn 4 des Ultraschallwandlers und die mit ihm verschraubte Metallsonde 7 zweckmäßig aus einem Material mit einer im wesentlichen gleichen akustischen Impedanz bestehen. Als bevorzugte Materialien kommen Edelstahl und Titan in Betracht. Weiterhin sollte das Horn 4 mit einer sich in axialer Richtung etwa auf den Querschnitt des Schraubansatzes 8 der Metallsonde 7 verjüngenden Hüllfläche in der Ausbildung einer Exponentialkurve versehen sein, wobei für die Hüllfläche auch andere Geometrien berücksichtigt werden können. Die mit den elastischen Mitteln 10, 11 bewirkte Abstützung des Ultraschallwandlers an dem umgebenden Gehäuse sollte derart ausgeführt sein, daß sowohl für die periodische wie auch für die impulsförmige Schwingungsanregung der Metallsonde jeweils optimale Arbeitsergebnisse erhalten werden. Für die Umschaltung zwischen der periodischen und der impulsförmigen Schwingungsanregung der Metallsonde kann auch eine zu dem Umschalter 21 zusätzliche Schaltereinrichtung vorgesehen werden, die zwischen getrennten Anschlußverbindungen des Ultraschallwandlers umschaltbar ist.

## Patentansprüche

1. Vorrichtung zum Entfernen von Körpersteinen mit einem intrakorporalen Lithotriper, bestehend aus:
- einer als Wellenleiter dienenden Metallsonde bzw. Sonotrode (7), die für eine mit ihrem distalen Ende veranlasste Steinzertrümmerung in den Arbeitskanal eines Endoskops eingesetzt wird; und
- einem elektrisch angesteuerten Ultraschallwandler, der die Metallsonde bzw. Sonotrode (7) zu longitudinalen Schwingungen anregt, **dadurch gekennzeichnet, dass**
- der Ultraschallwandler mit wenigstens einer piezokeramischen Scheibe (1, 2) gebildet ist, die mit einer Anordnung zwischen einem Reflektor (3) und einem die metallsonde bzw. Sonotrode (7) tragenden Horn (4) durch eine die Anordnung axial durchsetzende Spannschraube (5) gegeneinander verspannt sind, und
- die elektrische Ansteuerung des Ultraschallwandlers zwischen einer periodischen Schwingungsanregung der Metallsonde bzw. Sonotrode (7) und einer impulsförmigen Schwingungsanregung der Metallsonde bzw. Sonotrode (7) aufgrund eines einzigen Spannungsimpulses umschaltbar ist.

2. Vorrichtung nach Anspruch 1, **dadurch gekennzeichnet, dass** für impulsförmige und die periodische Schwingungsanregung der Metallsonde bzw. Sonotrode (7) zwei getrennte Schaltungsanordnungen (13, 14) vorgesehen sind, wobei
- eine erste Schaltungsanordnung (13) für die Steuerung der periodischen Schwingungsanregung der Metallsonde bzw. Sonotrode (7) mit einem spannungsgesteuerten Oszillator (15) gebildet ist, der über einen U/F-Wandler (16) und einen nachgeschalteten Verstärker (17) mit einem Impedanz-Transformator (18) verbunden ist, sowie mit einem Phasenkomparator (19), welcher zur Erzeugung einer Regelspannung für eine Steuerung des Oszillators (15) die Phasen der Ausgangsspannung und des Ausgangsstroms des Impedanz-Transformators (18) vergleicht,
- eine zweite Schaltungsanordnung (14) für die Steuerung der impulsförmigen Schwingungsanregung der Metallsonde bzw. Sonotrode (7) mit einer Spannungsquelle (22) und mit einem Kondensator (23) gebildet und über einen Umschalter (21) in eine Anschlussverbindung (20) mit dem Ultraschallwandler einschaltbar ist.

3. Vorrichtung nach Anspruch 2, **dadurch gekennzeichnet, dass** der Kondensator (23) der zweiten Schaltungsanordnung (14) in einer ersten Schaltstellung des Umschalters (21) durch die Spannungsquelle (22) aufgeladen wird und für die Anschlussverbindung (20) mit dem Ultraschallwandler in eine zweite Schaltstellung umschaltbar ist.

4. Vorrichtung nach einem der Ansprüche 1 bis 3, **dadurch gekennzeichnet, dass** die mit der Spannschraube (5) gegenseitig verspannte Anordnung des Reflektors (3), der wenigstens einen piezokeramischen Scheibe (1, 2) und des die Metallsonde bzw. Sonotrode (7) tragenden Horns (4) des Ultraschallwandlers durch elastische Stützmittel (10, 11) gegen ein umgebendes Gehäuse abgestützt ist.

5. Vorrichtung nach einem der Ansprüche 1 bis 4, **dadurch gekennzeichnet, dass** das Horn (4) des Ultraschallwandlers mit einer sich in axialer Richtung etwa auf den Querschnitt eines Schraubansatzes (8) der Metallsonde bzw. Sonotrode (7) verjüngenden Hüllfläche in der Ausbildung einer Exponentialkurve versehen ist.

6. Vorrichtung nach einem der Ansprüche 1 bis 5, **dadurch gekennzeichnet, dass** die Spannschraube (5) hohl ausgebildet ist für den Anschluss eines äußeren Sauganschlusses (12) an eine mit dem Horn (4) des Ultraschallwandlers verschraubte und mit dem Hohlraum der Spannschraube (5) axial fluchtende rohrförmige Metallsonde bzw. Sonotrode (7).

7. Vorrichtung nach einem der Ansprüche 1 bis 6, **dadurch gekennzeichnet, dass** die Metallsonde bzw. Sonotrode (7) und das Horn (4) des Ultraschallwandlers aus einem Material mit einer im wesentlichen gleichen akustischen Impedanz bestehen.

8. Vorrichtung nach einem der Ansprüche 1 bis 7, **dadurch gekennzeichnet, dass** für die Umschaltung zwischen der periodischen und der impulsförmigen Schwingungsanregung der Metallsonde bzw. Sonotrode (7) eine separate Schalteinrichtung zwischen getrennten Anschlussverbindungen (20) an den Ultraschallwandler vorgesehen ist.

## Claims

1. A device for removing calculi by using an intracorporeal lithotripter, comprising:
- a metal probe or sonotrode (7) acting as a waveguide and introduced into a working channel of an endoscope to cause, by its distal end, a fragmentation of stones or calculi; and
- an electrically controlled ultrasonic transducer for stimulating the metal probe or sonotrode (7) to generate longitudinal oscillations, **characterized in that**
- the ultrasonic transducer is formed with at least one piezo-ceramic disc (1, 2) arranged between a reflector (3) and a horn (4) carrying said metal probe or sonotrode (7), the arrangement being fixedly interconnected by an axially traversing clamping screw (5), and
- the electric controlling of the ultrasonic transducer is switchable by one single voltage pulse between a periodically stimulated oscillation of said metal probe or sonotrode (7) and a pulse wave stimulated oscillation of said metal probe or sonotrode (7).

2. The device according to claim 1, **characterized in that** two separate circuit arrangements (13, 14) are provided for said pulse wave and said periodically stimulated oscillations of said metal probe or sonotrode (7), wherein
- a first circuit arrangement (13) for controlling of the periodically stimulated oscillation of said metal probe or sonotrode (7) is formed with a voltage-controlled oscillator (15) being connected via a voltage-to-frequency converter (16) and a downstream amplifier (17) to an impedance transformer (18), and with a phase comparator (19) which is arranged for comparing the phases of the output voltage and the output current of the impedance transformer (18) to thereby generate a control voltage for the controlling of the oscillator (15),
- a second circuit arrangement (14) for controlling the pulse wave stimulated oscillation of said metal probe or sonotrode (7) being formed with a voltage source (22) and a capacitor (23), and switched on via a change-over switch (21) to an interconnection (20) to the ultrasonic transducer.

3. The device according to claim 2, **characterized in that** said capacitor (23) of said second circuit arrangement (14) is loaded by said voltage source (22) in a first switching position of said change-over switch (21), and can be switched into a second switching position for said interconnection (20) to the ultrasonic transducer.

4. The device according to any one of claims 1 to 3, **characterized in that** the arrangement of said reflector (3), which is fixedly interconnected by said clamping screw (5), said at least one piezo-ceramic disc (1, 2) and said horn (4), that carries said metal probe or sonotrode (7), of the ultrasonic transducer is supported against a surrounding casing by elastic support means (10,11).

5. The device according to any one of claims 1 to 4, **characterized in that** the horn (4) of the ultrasonic transducer has an enveloping surface forming an exponential curve, which tapers in the axial direction substantially to the cross-section of a screw connection (8) of said metal probe or sonotrode (7).

6. The device according to any one of claims 1 to 5, **characterized in that** the clamping screw (5) is hollow to thereby interconnect an outer suction connection (12) with a tubular metal probe or sonotrode (7) which is screwed to the horn (4) of the ultrasonic transducer and is axially aligned with the hollow space of the clamping screw (5).

7. The device according to any one of claims 1 to 6, **characterized in that** the metal probe or sonotrode (7) and the horn (4) of the ultrasonic transducer consist of a material with substantially the same acoustic impedance.

8. The device according to any one of claims 1 to 7, **characterized in that** for switching between the periodic and the pulse wave stimulated oscillation of said metal probe or sonotrode (7), a separate switch unit is provided between separate interconnections (20) to the ultrasonic transducer.

## Revendications

1. Appareil pour supprimer des calculs corporels avec un lithotripteur intracorporel, comprenant :
- une sonde métallique ou sonotrode (7) servant de guide d'onde, qui est employée dans le canal de travail d'un endoscope pour le broyage des calculs, provoqué par son extrémité distale ; et
- un convertisseur à ultrasons à pilotage électrique, qui excite la sonde métallique ou la sonotrode (7) pour des oscillations longitudinales,
**caractérisé en ce que**
- le convertisseur à ultrasons est formé avec au moins une plaque piézocéramique (1, 2), laquelle est serrée avec un agencement entre un réflecteur (3) et un cornet (4) qui porte la sonde métallique ou la sonotrode (7) au moyen d'une vis de serrage (5) qui traverse axialement l'agencement, et
le pilotage électrique du convertisseur à ultrasons peut être commuté entre une excitation périodique des oscillations de la sonde métallique ou de la sonotrode (7), et une excitation par impulsions des oscillations de la sonde métallique ou de la sonotrode (7) à la suite d'une unique impulsion de tension.

2. Appareil selon la revendication 1, **caractérisé en ce que** pour l'excitation périodique et pour l'excitation par impulsions des oscillations de la sonde métallique ou de la sonotrode (7), sont prévus deux agencements de circuits séparés (13, 14), dans lesquels
- un premier agencement de circuit (13) est formé pour le pilotage de l'excitation périodique des oscillations de la sonde métallique ou de la sonotrode (7) avec un oscillateur commandé en tension (15), lequel est relié à un transformateur d'impédance (18) via un convertisseur U/F (16) et un amplificateur (17) branché à la suite, ainsi qu'avec un comparateur de phase (19) qui, pour générer une tension de régulation pour une commande de l'oscillateur (15), compare les phases de la tension de sortie et du courant de sortie du transformateur d'impédance (18),
- un second agencement de circuit (14) est formé pour la commande de l'excitation par impulsions des oscillations de la sonde métallique ou de la sonotrode (7) avec une source de tension (22) et avec un condensateur, et qui peut être mis en circuit via un inverseur (21) dans une liaison de raccordement (20) avec le convertisseur à ultrasons.

3. Appareil selon la revendication 2, **caractérisé en ce que** le condensateur (23) du second agencement de circuit (14) est chargé par la source de tension (22) dans une première position de commutation de l'inverseur (21), et peut être inversé dans une seconde position de commutation pour la liaison de raccordement (20) avec le convertisseur à ultrasons.

4. Appareil selon l'une des revendications 1 à 3, **caractérisé en ce que** l'agencement, mutuellement serré avec la vis de serrage (5), formé du réflecteur (3), de ladite au moins une plaque piézocéramique (1, 2), et du cornet (4) du convertisseur à ultrasons qui porte la sonde métallique ou la sonotrode (7), est soutenu par des moyens de soutien élastiques (10, 11) contre un boîtier périphérique.

5. Appareil selon l'une des revendications 1 à 4, **caractérisé en ce que** le cornet (4) du convertisseur à ultrasons est doté d'une surface enveloppe qui va en se rétrécissant en direction axiale approximativement jusqu'à la section d'un talon de vissage (8) de la sonde métallique ou de la sonotrode (7), réalisée sous la forme d'une courbe exponentielle.

6. Appareil selon l'une des revendications 1 à 5, **caractérisé en ce que** la vis de serrage (5) est réalisée creuse pour le raccordement d'un raccord d'aspiration extérieur (12) à une sonde métallique ou une sonotrode (7) de forme tubulaire vissée avec le cornet (4) du convertisseur à ultrasons et en alignement axial avec la cavité de la vis de serrage (5).

7. Appareil selon l'une des revendications 1 à 6, **caractérisé en ce que** la sonde métallique ou la sonotrode (7) et le cornet (4) du convertisseur à ultrasons sont en un matériau qui présente essentiellement la même impédance acoustique.

8. Appareil selon l'une des revendications 1 à 7, **caractérisé en ce que**, pour l'inversion entre l'excitation périodique et l'excitation par impulsions des oscillations de la sonde métallique ou de la sonotrode (7) il est prévu un dispositif de commutation séparé entre des liaisons de raccordement séparées (20) vers le convertisseur à ultrasons.
